**Europäisches Patentamt**

**European Patent Office**

**Office européen des brevets**

(19)

(11) Veröffentlichungsnummer : **0 122 539**
**B1**

# (12) EUROPÄISCHE PATENTSCHRIFT

(45) Veröffentlichungstag der Patentschrift :
30.09.87

(21) Anmeldenummer : **84103689.0**

(22) Anmeldetag : **04.04.84**

(51) Int. Cl.⁴ : **C 07 C 29/86, C 07 C 31/08**

(54) **Verfahren zur Abtrennung von Äthanol aus einer äthanolhaltigen Lösung.**

(30) Priorität : **14.04.83 DE 3313530**

(43) Veröffentlichungstag der Anmeldung :
**24.10.84 Patentblatt 84/43**

(45) Bekanntmachung des Hinweises auf die Patenterteilung : **30.09.87 Patentblatt 87/40**

(84) Benannte Vertragsstaaten :
**AT CH FR GB IT LI**

(56) Entgegenhaltungen :
**GB-A- 2 059 787**
**US-A- 4 277 635**

(73) Patentinhaber : **Fried. Krupp Gesellschaft mit beschränkter Haftung**
**Altendorfer Strasse 103**
**D-4300 Essen 1 (DE)**

(72) Erfinder : **Hagen, Rainer**
**Schäferstrasse 24**
**D-4300 Essen 1 (DE)**
Erfinder : **Hartwig, Jürgen, Dr.**
**Büssemstrasse 24**
**D-4300 Essen 14 (DE)**

**Beschreibung**

Die Erfindung bezieht sich auf ein Verfahren zur Abtrennung von Äthanol aus der bei der alkoholischen Gärung anfallenden äthanolhaltigen Lösung, bei dem die äthanolhaltige Lösung mit einem Lösungsmittel extrahiert wird, welches bei 20 °C und 1 bar gasförmig ist und sich während der Extraktion im flüssigen oder überkritischen Zustand befindet, und bei dem die äthanolhaltige Lösungsmittelphase von der äthanolarmen Lösung getrennt sowie danach zu Abtrennung des Äthanols in ihre Bestandteile zerlegt wird.

Bei der alkoholischen Gärung werden Zucker, insbesondere Glucose in Gegenwart von Hefepilzen, die den Enzymkomplex Zymase enthalten, zu Äthanol und Kohlendioxid abgebaut. Die Glucose wird durch enzymatische Spaltung der Maltose erzeugt, die wiederum bei der hydrolytisch-enzymatischen Spaltung von Stärke gebildet wird oder bei der Zuckerfabrikation anfällt. Die bei der alkoholischen Gärung anfallenden äthanolhaltigen Lösungen enthalten neben dem Äthanol lösliche und unlösliche Bestandteile pflanzlicher Zellen und Gerüstsubstanzen, Hefezellen, Stärke und Bruchstücke der Stärke, verschiedene Zucker, Salze und Wasser. Der Äthanolgehalt der bei der alkoholischen Gärung anfallenden Lösungen beträgt üblicherweise ca. 6 Gew.-%, da durch eine höhere Äthanolkonzentration die Reaktionsgeschwindigkeit der alkoholischen Gärung zu stark herabgesetzt würde.

Äthanol kann aus den bei der alkoholischen Gärung anfallenden Lösungen durch Destillationsverfahren abgetrennt und bis zu einer Reinheit von 99,9 Gew.-% aufkonzentriert werden. Die Destillationsverfahren haben den Nachteil, daß sie sehr viel Energie benötigen. Allein die Destillation bis auf eine Äthanolkonzentration von 95 Gew.-% erfordert etwa 8 000 kJ/kg Destillat, während der Heizwert des Äthanols 26 780 kJ/kg beträgt. Für die Aufkonzentrierung über den azeotropen Punkt hinaus, der für 1 bar bei 95,58 Gew.-% Äthanol liegt, wird nochmals eine erhebliche Energiemenge benötigt. Der hohe Energieaufwand für die destillative Äthanolabtrennung ist ein wirtschaftliches Hindernis für die Verwendung des durch alkoholische Gärung erzeugten Äthanols als Motortreibstoff.

Um den hohen Energieaufwand für die destillative Äthanolabtrennung zu vermeiden, kann Äthanol durch Extraktion mit einem Lösungsmittel aus seinen Lösungen abgetrennt werden. Aus der DE-OS 3 033 729 ist ein Verfahren zur Abtrennung von Äthanol aus einer wäßrigen Lösung bekannt, bei dem die äthanolhaltige Lösung mit flüssigem oder überkritischem $CO_2$, $C_2H_4$ oder $C_2H_6$ extrahiert wird. Bei Verwendung von $CO_2$ als Extraktionsmittel kann die Extraktion bei 30 bis 150 atm und 0 bis 150 °C erfolgen. Bei diesem bekannten Verfahren wird der Druck der äthanolhaltigen Extraktphase reduziert und das Äthanol durch destillative Methoden vom Extraktionsmittel abgetrennt.

Auch aus der GB-A 20 59 787 ist schon ein Verfahren zu Extraktion von flüssigen organischen Komponenten aus einem Gemisch organischer Flüssigkeiten und Wasser mit überkritischen Lösungsmitteln bekannt geworden, bei dem an die Extraktion die Abscheidung der extrahierten Produkte aus der Lösungsmittelphase durch Druckabsenkung und die Fraktionierung der Extratphase durch nachgeschaltete Destillation erfolgt. Für die destillative Aufarbeitung der äthanolhaltigen Extraktphase wird eine erhebliche Energiemenge sowie ein großer apparativer Aufwand benötigt.

Gleiches gilt für das in der US-PS 4 277 635 beschriebene Verfahren zur Gewinnung und Aufkonzentrierung von Äthanol aus einer 6 bis 10 %-igen äthanolhaltigen Flüssigkeit bei der diese über entsprechende Adsorptionsmittelbetten geleitet und das Äthanol ausgeschieden wird.

Daher liegt der Erfindung die Aufgabe zugrunde, ein Verfahren zur Abtrennung von Äthanol aus der bei der alkoholischen Gärung anfallenden äthanolhaltigen Lösung durch Extraktion mit einem Lösungsmittel zu schaffen, das einen möglichst niedrigen Energieaufwand bei geringem apparativen Aufwand erfordert.

Die der Erfindung zugrundeliegende Aufgabe wird dadurch gelöst, daß die äthanolhaltige Lösungsmittelphase ohne Druck- und Temperaturänderung über ein Adsorptionsmittel geleitet wird, daß die äthanolfreie Lösungsmittelphase in die Extraktionsstufe zurückgeführt wird, daß durch Behandlung des äthanolhaltigen Adsorptionsmittels mit dem zur Extraktion verwendeten Lösungsmittel bei einem Druck von 1 bis 30 bar und einer Temperatur von 150 bis 300 °C das Äthanol ausgetrieben wird, daß die äthanolhaltige Gasphase durch Kühlung unter die dem gewählten Druck entsprechende Siedetemperatur des Äthanols in eine flüssige Äthanolphase sowie eine weitgehend äthanolfreie Gasphase getrennt wird und daß die weitgehend äthanolfreie Gasphase im Kreislaufgeführt wird. Dabei wird als Lösungsmittel $C_2H_4$, $C_2H_6$, $C_3H_6$, $C_3H_8$, $C_4H_8$, $C_4H_{10}$, LPG (Liquefied Petroleum Gas), $CO_2$, $CF_3Cl$, $CF_3Br$, $C_2F_2Cl_2H_2$, $N_2O$ verwendet und die Extraktion des Äthanols aus der äthanolhaltigen Lösung und die Adsorption des Äthanols aus der äthanolhaltigen Lösungsmittelphase bei einer Temperatur von 0 bis 200 °C und einem Druck von 1 bis 500 bar durchgeführt. Nach dem erfindungsgemäßen Verfahren kann Äthanol mit einem Energieaufwand aus der bei der alkoholischen Gärung anfallenden Lösung gewonnen werden, der etwa 50 % des Energieaufwands für eine atmosphärische Destillation des Äthanols auf eine Äthanol-Konzentration von 95 Gew.-% beträgt und der niedriger ist als der Energieaufwand, welcher zur destillativen Auftrennung einer überkritischen äthanolhaltigen Gasphase benötigt wird. Ein weiterer Vorteil des erfindungsgemäßen Verfahrens besteht darin, daß die Energiebilanz nicht durch das Auftreten azeotroper Gemische nachteilig beeinflußt wird. Es ist überraschend, daß Äthanol aus der Lösungsmittelphase durch ein Adsorptionsmittel bei den während der Extraktion der äthanolhaltigen Lösung herrschenden Druck- und Temperaturbedingungen abgetrennt werden kann, denn normalerweise werden flüssige oder überkritische Gase dazu benutzt, um beladene Adsorptionsmittel zu regenerieren, also die adsorbierten Stoffe von den Adsorptionsmitteln zu trennen (siehe DE-OS 25 44 116).

Vorteilhaft ist es, wenn die Extraktion des Äthanols aus der äthanolhaltigen Lösung und die Adsorption des Äthanols aus der äthanolhaltigen Lösungsmittelphase bei einer Temperatur von 20 bis 100 °C und einem Druck von 20 bis 250 bar durchgeführt wird und auch als Lösungsmittel ein Gemisch aus mindestens zwei der als Lösungsmittel genannten Stoffe verwendet werden. Es hat sich gezeigt, daß das Äthanol bei diesen Verfahrensbedingungen verteilhaft aus der äthanolhaltigen Lösung gewonnen werden kann. Nach der Erfindung ist ferner vorgesehen, daß als Adsorptionsmittel Aktivkohle, Kieselerde, Magnesiumoxid, Silicagel oder Zeolithe, vorzugsweise Aktivkohle, in granulierter oder pelletierter Form verwendet werden, da mit diesen Adsorptionsmitteln eine weitgehende Abtrennung des Äthanols aus der äthanolhaltigen Lösungsmittelphase erfolgt. Eine weitgehend quantitative Abtrennung des Äthanols aus dem Adsorptionsmittel ist nach der Erfindung dann gegeben, wenn 1 kg äthanolhaltiges Adsorptionsmittel mit 3 bis 25 kg, vorzugsweise 7 bis 20 kg, Lösungsmittel behandelt wird.

Eine besonders vorteilhafte, kontinuierliche Durchführung der alkoholischen Gärung und des erfindungsgemäßen Verfahrens ist dann möglich, wenn ein Teilstrom der bei der alkoholischen Gärung anfallenden äthanolhaltigen Lösung durch Sedimentation oder Zentrifugation von den Hefezellen befreit wird, wenn der hefezellenfreie Teilstrom extrahiert wird und wenn sowohl die abgetrennten Hefezellen als auch der äthanolarme Teilstrom in die alkoholische Gärung zurückgeführt wird. Nach der Erfindung kann die Abtrennung der Hefezellen vor der Extraktion der äthanolhaltigen Lösung dann entfallen, wenn ein Teilstrom der bei der alkoholischen Gärung anfallenden äthanolhaltigen Lösung bei 0 bis 38 °C extrahiert und anschließend der alkoholischen Gärung wieder zugeführt wird.

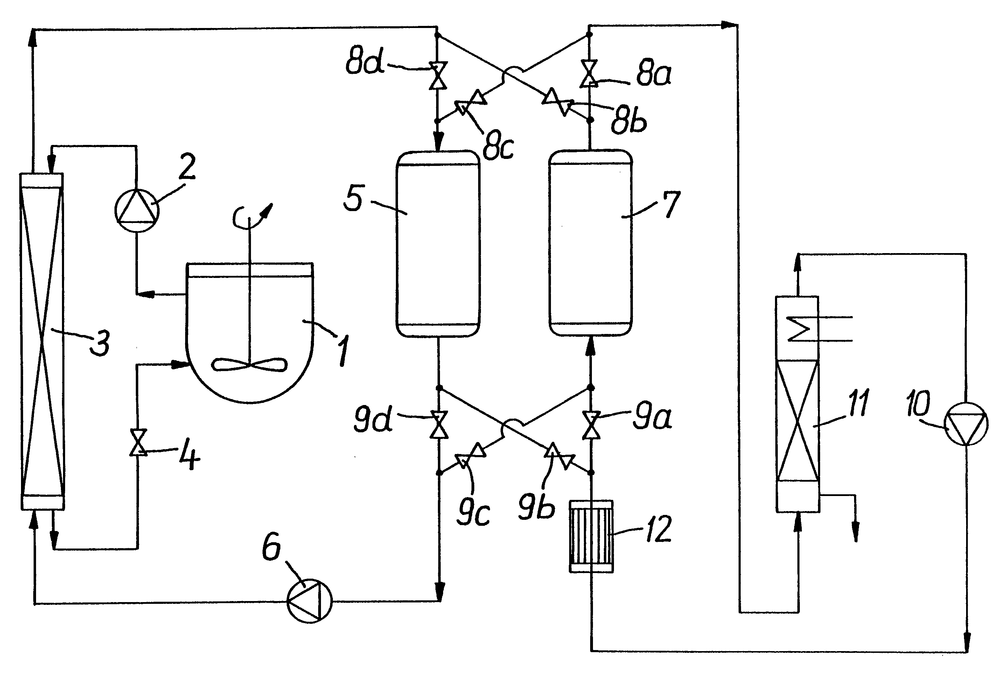

Der Gegenstand der Erfindung wird nachfolgend anhand eines Ausführungsbeispiels und der Zeichnung näher erläutert.

Ein Teilstrom der bei der alkoholischen Gärung anfallenden Lösung wird dem Fermenter 1 kontinuierlich entnommen, mit der Pumpe 2 auf den Extraktionsdruck gebracht und kopfseitig in die Stoffaustauschkolonne 3 gefördert. In der Stoffaustauschkolonne 3 wird die äthanolhaltige Lösung mit dem Lösungsmittel im Gegenstrom extrahiert. Als Lösungsmittel wird $C_2H_4$, $C_2H_6$,

$C_3H_6$, $C_3H_8$, $C_4H_8$, $C_4H_{10}$, LPG (liquefied petroleum gas = $C_3$-, $C_4$-, $C_5$-Kohlenwasserstoffe), $CO_2$, $CF_3Cl$, $CF_3Br$, $C_2F_2Cl_2H_2$, $N_2O$ oder ein Gemisch aus mindestens zwei dieser Stoffe verwendet. Besonders gut eignen sich die Lösungsmittel $C_2H_4$, $C_3H_6$, LPG oder $CO_2$. Die Extraktion der äthanolhaltigen Lösung wird bei einer Temperatur von 0 bis 200 °C, vorzugsweise 20 bis 100 °C, und einem Druck von 1 bis 500 bar, vorzugsweise 20 bis 250 bar, durchgeführt, wobei das Gewichtsverhältnis von Lösung zu Lösungsmittel 1 : 3 bis 1 : 10 beträgt.

Die an Äthanol abgereicherte Lösung wird am Boden der Stoffaustauschkolonne 3 abgezogen, über ein Druckreduzierventil 4 auf den Betriebsdruck des Fermenters entspannt und in diesen zurückgeführt. Dem Druckreduzierventil 4 kann noch ein in der Zeichnung nicht dargestellter Abscheidebehälter nachgeschaltet werden, in dem das gasförmige Lösungsmittel von der äthanolarmen Lösung abgetrennt wird. Das gasförmige Lösungsmittel kann in den Extraktionskreislauf zurückgeführt werden. Die äthanolarme Lösung kann nach der Entgasung nicht nur in den Fermenter zurückgeführt, sondern auch zum Anmaischen des stärke- oder zuckerhaltigen Ausgangsmaterials verwendet werden.

Die äthanolhaltige Lösungsmittelphase verläßt die Stoffaustauschkolonne 3 über Kopf und strömt ohne Druck- und Temperaturänderung durch den Behälter 5, der mit einer Schüttung aus einem Adsorptionsmittel gefüllt ist, das Äthanol aufnimmt. Die Adsorptionsmittelschüttung hat einen geringen Strömungswiderstand, da das Adsorptionsmittel in granulierter oder pelletierter Form eingesetzt wird. Nach dem Verlassen des Behälters 5 ist die Lösungsmittelphase annähernd äthanolfrei, und sie wird vom Kompressor 6 in die Stoffaustauschkolonne 3 gefördert. Sobald das im Behälter 5 vorhandene Adsorptionsmittel mit Äthanol beladen ist, wird der Behälter 5 durch eine entsprechende Schaltung der Absperrventile 8a bis 8d und 9a bis 9d aus dem Extraktionskreislauf herausgenommen, und die äthanolhaltige Lösungsmittelphase wird dann in den Behälter 7 geführt, der eine frisch regenerierte Adsorptionsmittelschüttung enthält, die sich mit Äthanol belädt.

Nachdem das im Behälter 5 befindliche Adsorptionsmittel mit Äthanol gesättigt und der Behälter 5 aus dem Extraktionskreislauf herausgenommen worden ist, wird in den Behälter 5 überhitztes gasförmiges Lösungsmittel eingeleitet, das im Wärmeaustauscher 12 auf die Desorptionstemperatur aufgeheizt wurde und das adsorbierte Äthanol sowie geringe Wassermengen aufnimmt. Das äthanolhaltige gasförmige Lösungsmittel wird in der Kolonne 11 durch Kühlung unter die dem gewählten Druck entsprechende Siedetemperatur des Äthanols in die Komponenten getrennt. Am Fuß der Kolonne 11 wird flüssiges Äthanol abgenommen, während das weitgehend äthanolfreie gasförmige Lösungsmittel dem Wärmeaustauscher 12 vom Kompressor 10 zugeführt wird. Dadurch, daß zur Extraktion der äthanolhaltigen Lösung und zur Desorption des Äthanols der gleiche Stoff verwendet wird, ist es nicht nötig, das Adsorptionsmittel zu trocknen.

Es ist möglich, die Stoffaustauschkolonne 3 um einen in der Zeichnung nicht dargestellten Verstärkerteil zu erweitern, der mit einem Rücklauf aus wasserhaltigem Äthanol betrieben wird, das dem Regenerierungskreislauf entstammt. Der Verstärkerteil wird dann verwendet, wenn die äthanolhaltige Lösungsmittelphase einen zu hohen Wassergehalt aufweist. Der Rücklauf im Verstärkerteil muß soviel Wasser enthalten, daß er mit der äthanolhaltigen Lösungsmittelphase nicht vollständig mischbar ist.

Dem Fermenter 1 wird ein Teilstrom mit einem Äthanolgehalt von 5,6 Gew.-% und einer Temperatur von 38 °C entnommen und in einem Sedimentationstank von Hefezellen und anderen Feststoffen weitgehend befreit. Der hefehaltige Schlamm wird am Boden des Tanks abgezogen und in den Fermenter 1 zurückgeführt. Die äthanolhaltige, annähernd hefefreie Lösung wird auf 75 °C erwärmt sowie von der Pumpe 2 auf einen Druck von 80 bar komprimiert und in die Stoffaustauschkolonne 3 gefördert, wo sie im Gegenstrom zur aufsteigenden überkritischen $CO_2$-Phase geführt wird. Dabei geht Äthanol von der wäßrigen Phase in die überkritische $CO_2$-Phase über. Am Boden der Stoffaustauschkolonne 3 wird die auf eine Äthanolkonzentration von 2,2 Gew.-% abgereicherte Lösung abgezogen, die auf Atmosphärendruck entspannt, entgast und in die alkoholische Gärung zurückgeführt wird. Die Rückführung der äthanolarmen Lösung kann in der Weise erfolgen, daß sie entweder dem Fermenter 1 zugeführt oder zum Anmaischen des stärke- oder zuckerhaltigen Ausgangsmaterials verwendet wird.

Die äthanolhaltige Lösungsmittelphase gelangt vom Kopf der Stoffaustauschkolonne 3 in den Behälter 5 und strömt dort bei 75 °C und 80 bar über eine Schüttung aus granulierter Aktivkohle. Dabei wird Äthanol von der Aktivkohle adsorbiert, während das $CO_2$ den Behälter 5 verläßt und mit Hilfe des Kompressors 6 in den Sumpf der Stoffaustauschkolonne 3 zurückgeführt wird. Sobald die Aktivkohle mit Äthanol gesättigt ist, wird sie bei 200 °C und 10 bar mit einem $CO_2$-Strom behandelt, der das Äthanol sowie das restliche Wasser aufnimmt. Das $CO_2$-Äthanol-Gemisch gelangt in die Kolonne 11, in der es auf 75 °C abgekühlt wird, wobei das Äthanol in flüssiger Form anfällt und im Kolonnensumpf mit einem Restwassergehalt von 1,5 Gew.-% abgezogen wird. Das $CO_2$ verläßt die Kolonne 11 über Kopf und wird im Wärmeaustauscher 12 auf 200 °C aufgeheizt sowie vom Kompressor 10 in den Behälter 5 bzw. den Behälter 7 zurückgeführt. Bei der Extraktion beträgt das Mengenverhältnis zwischen $CO_2$ und der äthanolhaltigen Lösung 7 kg $CO_2$/kg Lösung. Zum Regenerieren von 1 kg Aktivkohle werden 7,1 kg $CO_2$ benötigt.

**Patentansprüche**

1. Verfahren zur Abtrennung von Äthanol aus der bei der alkoholischen Gärung anfallenden äthanolhaltigen Lösung, bei dem die äthanolhaltige Lösung mit einem Lösungsmittel extrahiert wird, welches bei 20 °C und 1 bar gasförmig ist und sich während der Extraktion im flüssigen oder überkritischen Zustand befindet, und bei dem die äthanolhaltige Lösungsmittelphase von der äthanolarmen Lösung getrennt sowie danach zu Abtrennung des Äthanols in ihre Bestandteile zerlegt wird, dadurch gekennzeichnet, daß die äthanolhaltige Lösungsmittelphase ohne Druck- und Temperaturänderung über ein Adsorptionsmittel geleitet wird, daß die äthanolfreie Lösungsmittelphase in die Extraktionsstufe zurückgeführt wird, daß durch Behandlung des äthanolhaltigen Adsorptionsmittels mit dem zur Extraktion verwendeten Lösungsmittel bei einem Druck von 1 bis 30 bar und einer Temperatur von 150 bis 300 °C das Äthanol ausgetrieben wird, daß die äthanolhaltige Gasphase durch Kühlung unter die dem gewählten Druck entsprechende Siedetemperatur des Äthanols in eine flüssige Äthanolphase sowie eine weitgehend äthanolfreie Gasphase getrennt wird und daß die weitgehend äthanolfreie Gasphase im Kreislauf geführt wird, wobei als Lösungsmittel $C_2H_4$, $C_2H_6$, $C_3H_6$, $C_3H_8$, $C_4H_8$, $C_4H_{10}$, LPG (Liquefied Petroleum Gas), $CO_2$, $CF_3Cl$, $CF_3Br$, $C_2F_2Cl_2H_2$ oder $N_2O$ verwendet wird und die Extraktion des Äthanols aus der äthanolhaltigen Lösung und die Adsorption des Äthanols aus der äthanolhaltigen Lösungsmittelphase bei einer Temperatur von 0 bis 200 °C und einem Druck von 1 bis 500 bar durchgeführt wird.

2. Verfahren nach Anspruch 1, dadurch gekennzeichnet, daß die Extraktion des Äthanols aus der äthanolhaltigen Lösung und die Adsorption des Äthanols aus der äthanolhaltigen Lösungsmittelphase bei einer Temperatur von 20 bis 100 °C und einem Druck von 20 bis 250 bar durchgeführt wird.

3. Verfahren nach Ansprüchen 1 und 2, dadurch gekennzeichnet, daß als Lösungsmittel ein Gemisch von mindestens zwei der im Anspruch 1 als Lösungsmittel genannten Stoffe verwendet wird.

4. Verfahren nach den Ansprüchen 1 bis 3, dadurch gekennzeichnet, daß als Adsorptionsmittel Aktivkohle, Kieselerde, Magnesiumoxid, Silicagel oder Zeolithe, vorzugsweise Aktivkohle, in granulierter oder pelletierter Form verwendet werden.

5. Verfahren nach den Ansprüchen 1 bis 4, dadurch gekennzeichnet, daß 1 kg äthanolhaltiges Adsorptionsmittel mit 3 bis 25 kg, vorzugsweise 7 bis 20 kg, Lösungsmittel behandelt wird.

6. Verfahren nach den Ansprüchen 1 bis 5, dadurch gekennzeichnet, daß ein Teilstrom der bei der alkoholischen Gärung anfallenden äthanolhaltigen Lösung durch Sedimentation oder Zentrifugation von den Hefezellen befreit wird, daß der hefezellenfreie Teilstrom extrahiert wird und daß sowohl die abgetrennten Hefezellen als auch der äthanolarme Teilstrom in die alkoholische Gärung zurückgeführt werden.

7. Verfahren nach den Ansprüchen 1 bis 5, dadurch gekennzeichnet, daß ein Teilstrom der bei der alkoholischen Gärung anfallenden äthanolhaltigen Lösung bei 0 bis 38 °C extrahiert und anschließend der alkoholischen Gärung wieder zugeführt wird.

### Claims

1. A process for the separation of ethanol from the ethanol-containing solution occurring during alcoholic fermentation, wherein the ethanol-containing solution is extracted with a solvent which is gaseous at 20 °C and 1 bar and is in the liquid or supercritical state during extraction, and wherein the ethanol-containing solvent phase is separated from the low-ethanol solution and then broken down into its components to separate the ethanol, characterized in that the ethanol-containing solvent phase is passed without alteration of pressure or temperature over an adsorption agent ; the ethanol-free solvent phase is returned to the extraction stage ; the ethanol is expelled by treating the ethanol-containing adsorption agent with the solvent used for extraction at a pressure of 1 to 30 bar and a temperature of 150 to 300 °C; the ethanol-containing gaseous phase is separated by cooling below the ethanol boiling temperature corresponding to the pressure selected into a liquid ethanol phase and a substantially ethanol-free gaseous phase ; the substantially ethanol-free gaseous phase is circulated, the solvent used being $C_2H_4$, $C_2H_6$, $C_3H_6$, $C_3H_8$, $C_4H_8$, $C_4H_{10}$, LPG (liquefied petroleum gas), $CO_2$, $CF_3Cl$, $CF_3Br$, $C_2F_2Cl_2H_2$ or $N_2O$ ; and the ethanol is extracted from the ethanol-containing solution and the ethanol is adsorbed from the ethanol-containing solvent phase at a temperature of 0 to 200 °C and a pressure of 1 to 500 bar.

2. A process according to Claim 1, characterized in that the ethanol is extracted from the ethanol-containing solution and the ethanol is adsorbed from the ethanol-containing solvent phase at a temperature of 20 to 100 °C and a pressure of 20 to 250 bar.

3. A process according to Claims 1 and 2, characterized in that the solvent used is a mixture of at least two of the substances named as solvents in Claim 1.

4. A process according to Claims 1 to 3, characterized in that the adsorption agent used is activated charcoal, silica, magnesium oxide, silica gel or zeolites, preferably activated charcoal, in the form of granules or pellets.

5. A process according to Claims 1 to 4, characterized in that 1 kg of ethanol-containing adsorption agent is treated with 3 to 25 kg, preferably 10 to 20 kg of solvent.

6. A process according to Claims 1 to 5, characterized in that a component flow of the ethanol-containing solution occurring during alcoholic fermentation is freed from the yeast cells by sedimentation or centrifugation ; the yeast-cell-free component flow is extracted ; and both the separated yeast cells and the low-ethanol component flow are returned to alcoholic fermentation.

7. A process according to Claims 1 to 5, characterized in that a component flow of the ethanol-containing solution occurring during alcoholic fermentation is extracted at 0 to 38 °C and then returned to alcoholic fermentation.

### Revendications

1. Procédé pour la séparation de l'éthanol de la solution contenant de l'éthanol obtenue lors de la fermentation alcoolique, dans lequel on extrait la solution contenant de l'éthanol par un solvant qui est gazeux à 20 °C et 1 bar et se trouve, pendant l'extraction, dans l'état liquide ou hypercritique, et dans lequel on sépare la phase de solvant contenant de l'éthanol de la solution pauvre en éthanol, et ensuite, on la dissocie en ses constituants pour séparer l'éthanol, caractérisé par le fait que l'on fait passer la phase de solvant contenant de l'éthanol, sans variation de pression ni de température, sur un agent d'adsorption, que l'on ramène la phase de solvant exempte d'éthanol à l'étape d'extraction, que, par traitement de l'agent d'adsorption contenant de l'éthanol par le solvant utilisé pour l'extraction à une pression de 1 à 30 bar et à une température de 150 à 300 °C, on chasse l'éthanol, que l'on sépare la phase gazeuse contenant de l'éthanol, par refroidissement en dessous de la température d'ébullition de l'éthanol correspondant à la pression choisie, en une phase liquide d'éthanol et une phase gazeuse dans une large mesure exempte d'éthanol, et que l'on recycle la phase gazeuse dans une large mesure exempte d'éthanol en utilisant comme solvant $C_2H_4$, $C_2H_6$, $C_3H_6$, $C_3H_8$, $C_4H_8$, $C_4H_{10}$, le GPL (gaz de pétrole liquéfié), $CO_2$, $CF_3Cl$, $CF_3Br$, $C_2F_2Cl_2H_2$ ou $N_2O$, et en effectuant l'extraction de l'éthanol de la solution contenant de l'éthanol et l'adsorption de l'éthanol de la phase de solvant contenant de l'éthanol à une température de 0 à 200 °C et à une pression de 1 à 500 bar.

2. Procédé selon la revendication 1, caractérisé par le fait que l'on effectue l'extraction de l'éthanol de la solution contenant de l'éthanol et l'adsorption de l'éthanol de la phase de solvant contenant de l'éthanol à une température de 20 à 100 °C et à une pression de 20 à 250 bar.

3. Procédé selon les revendications 1 et 2, caractérisé par le fait que l'on utilise comme solvant un mélange d'au moins deux des corps indiqués comme solvants dans la revendication 1.

4. Procédé selon l'une des revendications 1 à 3, caractérisé en ce que l'on utilise comme agent d'adsorption le charbon activé, la silice, l'oxyde de magnésium, le gel de silice ou les zéolites, de préférence le charbon activé, sous forme granulée ou pastillée.

5. Procédé selon l'une des revendications 1 à 4, caractérisé en ce que l'on traite 1 kg d'agent d'adsorption contenant de l'éthanol par 3 à 25 kg, de préférence 7 à 20 kg de solvant.

6. Procédé selon l'une des revendications 1 à 5, caractérisé en ce que l'on débarrasse des cellules de levure, par sédimentation ou centrifugation, un courant partiel de la solution contenant de l'éthanol obtenue lors de la fermentation alcoolique, et que l'on ramène à la fermentation alcoolique aussi bien les cellules de levure séparées que le courant partiel pauvre en éthanol.

7. Procédé selon l'une des revendications 1 à 5, caractérisé en ce que l'on extrait, de 0 à 38 °C, un courant partiel de la solution contenant de l'éthanol obtenue lors de la fermentation alcoolique, et qu'ensuite on le ramène à la fermentation alcoolique.

0 122 539